# FASCICULE DE BREVET EUROPEEN

(11) **EP 2 109 440 B1**
(45) Date de publication et mention de la délivrance du brevet: **13.01.2016**
(21) Numéro de dépôt: 08761761.9
(22) Date de dépôt: 15.01.2008
(51) Int. Cl.: A61K 8/49, A61K 8/64, A61K 8/67, A61K 31/375, A61K 31/455, A61K 38/08, A61Q 19/02, A61P 19/02

(54) **ASSOCIATION DE COMPOSES INHIBITEURS DE LA MELANOGENESE ET LEURS UTILISATIONS EN COSMETIQUE ET EN DERMATOLOGIE.**
ASSOZIATION VON MELANOGENESEHEMMENDEN VERBINDUNGEN UND VERWENDUNG IN DER KOSMETIK UND DERMATOLOGIE
ASSOCIATION OF COMPOUNDS INHIBITING MELANOGENESIS AND USE THEREOF IN COSMETICS AND DERMATOLOGY

(30) Priorité: 16.01.2007 FR 0700279
(43) Date de publication de la demande: 21.10.2009
(73) Titulaire: LABORATOIRES MAYOLY SPINDLER, 78401 Chatou Cédex (FR)
(72) Inventeur: CRITON, Marc, F-34090 Montpellier (FR); LEMELLAY HAMON, Véronique, F-78500 Sartrouville (FR); LEBLOND, Yves, F-78630 Orgeval (FR)
(74) Mandataire: Corizzi, Valérie
(86) Numéro de dépôt international: PCT/FR2008/000040
(87) Numéro de publication internationale: WO 2008/107533

(56) Documents cités:
- WO-A-2006/102289
- IMAE K ET AL: "SYNTHESIS, STEREOCHEMISTRY, AND BIOLOGICAL PROPERTIES OF THE DEPIGMENTING AGENTS, MELANOSTATIN, FELDAMYCIN AND ANALOGS" JOURNAL OF ANTIBIOTICS, JAPAN ANTIBIOTICS RESEARCH ASSOCIATION, TOKYO, JP, vol. 44, no. 1, janvier 1991 (1991-01), pages 76-85, XP002030833 ISSN: 0021-8820
- ISHIHARA Y ET AL: "Melanostatin, a new melanin synthesis inhibitor. Production, isolation, chemical properties, structure and biological activity." THE JOURNAL OF ANTIBIOTICS JAN 1991, vol. 44, no. 1, janvier 1991 (1991-01), pages 25-32, XP009089534 ISSN: 0021-8820
- JAYAWICKREME E A: "Discovery and structure-function analysis of alpha-MSH antagonists", JOURNAL OF BIOLOGICAL CHEMISTRY, AMERICAN SOCIETY FOR BIOCHEMISTRY AND MOLECULAR BIOLOGY, US, vol. 269, no. 47, 25 November 1994 (1994-11-25), pages 29846-29854, XP002112308, ISSN: 0021-9258

## Description

L'invention a pour objet une nouvelle association de composés inhibiteurs de la mélanogénèse et ses utilisations en cosmétique et en dermatologie pour préparer des compositions dépigmentantes blanchissantes et/ou éclaircissantes.

Prisés par les pays asiatiques où la blancheur de la peau est un véritable critère esthétique mais également par les populations occidentales où l'homogénéité du teint est un signe de santé corporelle, les produits éclaircissants sont un véritable phénomène de société. Les secteurs de la dermatologie et de la cosmétique ont su répondre à ce besoin en proposant des produits dépigmentants, éclaircissants ou blanchissants destinés à favoriser l'élimination des taches pigmentaires (taches solaires, taches de rousseur, taches de sénescence) ou à éclaircir le teint.

La pigmentation cutanée et folliculaire est la résultante de l'exposition de mélanine à la surface de la peau et du follicule pileux. La mélanogénèse est assurée spécifiquement par les mélanocytes, cellules dendritiques présentes dans la couche basale de l'épiderme, qui émettent des ramifications de contact avec les kératinocytes. La mélanine nouvellement synthétisée est transférée des dendrites mélanocytaires vers les kératinocytes qui finalement exposent la mélanine à la surface de l'épiderme assurant ainsi une coloration uniforme de l'épiderme.

La synthèse des mélanines (phéomélanines, riches en soufre, donnant une couleur orangée ; eumélanines, conférant une couleur brune) est assurée au sein des mélanosomes, organelles apparentés aux lysosomes spécifiques des mélanocytes, par un processus enzymatique complexe. Trois enzymes localisées sur la face interne de la membrane mélanosomale sont impliquées successivement dans la mélanogénèse : la tyrosinase, la TRP-2 (tyrosinase-related protein-2) et la TRP-1 (tyrosinase-related protein-1). Précurseur de la synthèse de mélanine, la tyrosine est hydroxylée en Dopa (dihydroxyphénylalanine) puis oxydée en dopaquinone, ces deux transformations étant dues à l'action de la tyrosinase.

A ce stade, la synthèse de la mélanine peut être orientée vers la phéomélanine (mélanine jaune orangé) rencontrée chez les blonds ou les roux, ou vers l'eumélanine (mélanine brun foncé) rencontrée chez les personnes à pigmentation foncée. L'eumélanine résulte de la polymérisation de la dopaquinone pour conduire à la leucodopachrome puis à la dopachrome. Cette dernière est convertie à son tour soit en 5,6 dihydroxyindole (DHI) soit en acide 5,6-dihydroxyindole-2-carboxylique (DHICA) sous l'action de la TRP-2. A ce niveau, la synthèse de l'eumélanine peut se faire selon deux voies. La DHI est oxydée sous l'action de la tyrosinase ou d'une peroxydase en indole 5,6-quinone, tandis que la DHICA sous l'action de la TRP-1 conduit au 5,6-dihydroindole-2-carboxy acide. L'indole 5,6-quinone et le 5,6-dihydroindole-2-carboxyacide polymérisent pour former des mélanochromes puis de l'eumélanine.

La synthèse de la phéomélanine passe par la formation de composés soufrés (cystéinyl-DOPA) suite à l'action sur la dopaquinone du gluthation et de la cystéine. La cystéinyl-DOPA est transformée en alanyl-hydroxybenzothiazine, puis en phéomélanine.

Les mécanismes moléculaires régulant les mélanocytes et la production de mélanine sont relativement peu élucidés. Les travaux de Y. Yada ont montré que sous l'effet de rayonnements solaires de type UVB, les kératinocytes humains produisent et sécrètent l'hormone peptidique endothéline qui exerce un effet paracrine sur les mélanocytes (Imokawa G et al, J. Invest. Dermatol., 105 :32-37, 1995). L'endothéline active un récepteur membranaire (ETR) couplé à une protéine G induisant la prolifération des mélanocytes, la transcription des gènes codant pour la tyrosinase et l'ETR. De même, en réponse à des rayonnements UV, les kératinocytes et les mélanocytes sécrètent le peptide αMSH (hormone stimulante de la mélanocortine) qui régule l'activité de pigmentation des mélanocytes. Pour ce faire, l'αMSH se lie au MC-R (récepteur de la mélanocortine) induisant l'activation de la voie de transduction AMPc/PKA voire de la ser/thr kinase PKC aboutissant à la synthèse de novo de tyrosinase, et à la synthèse d'eumélanine. La PKCβ activerait directement la tyrosinase par phosphorylation de son domaine cytoplasmique (Park et al, J. Biol. Chem., 268 : 11742-11749, 1993). L'αMSH faciliterait également le transfert de mélanine vers les kératinocytes en stimulant la dendricité des mélanocytes (Hunt et al, J. Cell. Sci., 107 : 205-211, 1994).

Outre le souhait, pour certains individus ou populations, d'obtenir et de conserver un teint clair, se pose également pour beaucoup le problème de la prévention et du traitement d'hyperpigmentations localisées, sous formes de taches. Une hyperpigmentation localisée de la peau peut être d'origine endogène, comme c'est le cas des taches de rousseur, fréquentes chez les personnes à carnation claire. Elle peut également être le résultat d'une exposition aux UV. On observe une augmentation des taches de rousseur, dont la couleur fonce, sous l'effet des UV. On note également l'apparition de taches d'hyperpigmentation cutanée dans des zones soumises à une irritation (piqûre d'insecte, plaie à cicatrisation lente, eczéma, ...). Le facteur hormonal est à l'origine d'hyperpigmentations régionales par hyperactivité mélanocytaire telles que les mélasmas idiopathiques survenant lors de la grossesse (masque de grossesse) ou d'une contraception oestro-progestative. De même apparaissent souvent chez le sujet âgé des taches pigmentaires par hyperactivité et prolifération mélanocytaire bénigne (lentigo séniles).

Les substances connues pour leurs propriétés dépigmentantes peuvent agir selon l'un des mécanismes suivants :
- sur la viabilité des mélanocytes épidermiques et/ou folliculaires où se déroule la mélanogénèse,
- en interférant avec une des étapes de la biosynthèse de la mélanine, ou en inhibant une des enzymes impliquées dans la mélanogénèse, ou en s'intercalant comme analogue structural d'un des composés chimiques de la chaîne de synthèse de la mélanine,
- en interférant dans le transfert de la mélanine des mélanocytes vers les kératinocytes.

Les substances les plus utilisées sont pour la plupart des inhibiteurs de l'activité tyrosinase. On pourra citer les dérivés phénoliques. Ces dérivés ont une structure chimique comparable à celle de la tyrosine ou de la dopa et servent de substrat à la tyrosinase (inhibition compétitive). On trouve dans cette famille l'hydroquinone et ses dérivés. Toutefois ces produits présentent une cytotoxicité importante susceptible de provoquer des dépigmentations irréversibles et l'utilisation de l'hydroquinone dans les produits cosmétiques a été interdite par une réglementation européenne (Dir. 2000/6BC).

Diverses autres substances sont proposées comme dépigmentant. Certaines présentent une bonne tolérance locale mais aussi une efficacité réduite : la vitamine C, l'arbutine (hydroquinone β-D-gluconopyranoside), le niacinamide, qui agit sur le transfert des mélanosomes des mélanocytes vers les kératinocytes (Hakozaki T. et al., British Journal of Dermatology, 147 : 20-31, 2002), des extraits végétaux, notamment des extraits de soja (Paine C. et al., Journal of Investigative Dermatology, 116, 4 : 587-595 2001,) qui inhibent l'activité du récepteur PAR-2 (protease-activated receptor 2) exprimé par les kératinocytes.

Le document WO 2006/102289 décrit des compositions destinées au blanchiment de la peau. Ces compositions peuvent comprendre : un dérivé de vitamine C, du niacinamide, un extrait de concombre, un extrait de citron, un dérivé de phénylalanine tel que du SEPIWHITE®, un dérivé de créatinine.

Les documents K. Imae et al., The Journal of Antibiotics, 44(1), 1991, 76-85 et Y. Ishihara et al., The Journal of Antibiotics, 44(1), 1991, 25-32, décrivent une molécule nommée mélanostatine, inhibitrice de la synthèse de la mélanine, cette molécule étant un pseudo tripeptide.

La complexité des mécanismes impliqués dans la synthèse de la mélanine rend difficile le contrôle de la pigmentation de la peau dans des conditions satisfaisantes pour la santé des utilisateurs (Briganti S. et al., Pigment Cell Res., 16 : 101-110,2003).

On a donc cherché à mettre au point des compositions capables de prévenir et/ou de traiter l'apparition d'une hyperpigmentation de la peau et/ou des phanères, qu'il s'agisse d'une hyperpigmentation d'origine endogène ou d'origine exogène (UV, irritation cutanée, hormonale). On a également cherché à mettre au point des compositions qui ne soient pas toxiques et dont l'action soit réversible.

Notamment on a cherché à obtenir une bonne efficacité en vue de prévenir ou traiter les hyperpigmentations régionales dues à une hyperactivité mélanocytaire, telles que :
- les mélasmas idiopathiques, survenant lors de la grossesse, également appelés masque de grossesse ou chloasma, ou ceux qui sont la conséquence d'une contraception oestro-progestative ;
- les hyperpigmentations localisées par hyperactivité et prolifération mélanocytaire bénigne, telles que les taches de rousseur, les taches solaires ou les taches pigmentaires séniles, dites lentigo actiniques ;
- les hyperpigmentations ou dépigmentations accidentelles, éventuellement dues à la photosensibilisation ou à la cicatrisation post-lésionnelle, comme par exemple dans les zones soumises à une irritation (piqûre d'insecte, plaie à cicatrisation lente, eczéma, ...), ainsi que certaines leucodermies telles que le vitiligo.

On a également cherché à mettre au point des compositions ayant la propriété de blanchir la peau et/ou d'éclaircir le teint, et/ou d'uniformiser le teint, et/ou d'homogénéiser le teint.

La présente invention concerne une association comprenant au moins trois composés :
- un antagoniste du récepteur MC1-R,
- un inhibiteur de la tyrosinase dérivé de la vitamine C,
- un inhibiteur du transfert des mélanosomes vers les kératinocytes.

De façon surprenante, on a constaté que la combinaison de trois composés tels que définis ci-dessus aboutit à une inhibition supérieure à l'action isolée de ces 3 actifs. En outre, elle est dénuée d'effets toxiques et l'action combinée des trois actifs est réversible.

Par antagoniste du récepteur MC1-R on entend au sens de la présente invention un composé susceptible de se fixer sur les récepteurs cellulaires des mélanotropines (MC1-R) présents sur la membrane des mélanocytes, de bloquer la fixation de l'αMSH (Melanocortin Stimulating Hormone ou hormone stimulatrice de la mélanocortine), ligand spécifique du MC1-R, et d'inhiber l'activation du MC1-R par l'αMSH. Il existe deux antagonistes endogènes des MCR dont la protéine Agouti qui possède une forte affinité pour le MC1-R et intervient dans la régulation de la pigmentation cutanée (Suzuki I et al, J Invest Dermatol, 108: 838-842, 1997). Parmi les antagonistes du récepteur MC1-R connus de l'homme du métier on peut citer en particulier un oligopeptide découvert par l'Institut Européen de Biologie cellulaire (IEB), la MELANOSTATINE ^{®}5 (Nom INCI : nonapeptide-1), commercialisée par la société UNIPEX. Cet oligopeptide possède une affinité pour les récepteurs MC1-R et inhibe de façon spécifique et réversible la mélanogénèse en diminuant la synthèse et la production excessive de pigments de mélanine. Cet actif est par ailleurs dépourvu d'effets toxiques. On peut citer également le lipo amino acide undécylenoyl phénylalanine commercialisé par la société SEPPIC sous le nom de SEPIWHITE MSH ®.

Par inhibiteur de la tyrosinase dérivé de la vitamine C on entend au sens de la présente invention un composé choisi parmi les esters de l'acide ascorbique, comme par exemple l'acide 2-Glucoside Ascorbique (Nom INCI: Ascorbyl Glucoside), l'acide 2-O-alpha-D-glucopyranosyl-6-O-hexadecanoyl-L-ascorbique, le 6-palmitate d'ascorbyl, le sel de magnésium ou de sodium de l'acide 2-phosphate ascorbique. De préférence on utilise de l'acide 2-Glucoside Ascorbique, qui est commercialisé notamment par la société DKSH sous la marque AA-2G ®.

Par inhibiteur du transfert des mélanosomes, on entend au sens de la présente invention, un composé susceptible d'inhiber le transfert des mélanosomes vers les kératinocytes (Greatens A. et al. Exp Dermatol, 14: 498-508, 2005 ; Hakozaki T. et al Br. J Dermatol, 147: 20-31, 2002). En particulier, on entend par cette définition un composé tel que le Nicotinamide (Nom INCI : niacinamide), ou vitamine PP, qui est une des deux formes de la vitamine B3 et qui est commercialisé par la société MERCK.

La présente invention concerne donc une association constituée d'au moins trois actifs dénués d'effets toxiques agissant sur la mélanogénèse par trois mécanismes différents.

Etant donné la complexité des mécanismes de mélanogénèse, on s'attendait à ce que l'association de ces trois actifs ait un effet assez peu supérieur à celui du meilleur des trois. De façon surprenante, on a constaté que l'association de ces trois actifs conduisait à une inhibition de la mélanogénèse très supérieure au résultat attendu, qu'il s'agisse de la mélanogénèse endogène ou de celle qui est provoquée par un agent extérieur tel que des UV, et qu'en outre, cette inhibition était entièrement réversible.

La présente invention a également pour objet des compositions cosmétiques et/ou dermatologiques comprenant cette association dans un support cosmétiquement ou dermatologiquement acceptable, compatible avec une application sur la peau, les poils ou les cheveux.

Elle a également pour objet l'utilisation de cette association dans une composition cosmétique ou pour la préparation d'une composition dermatologique, en vue de prévenir ou traiter les hyperpigmentations régionales, et/ou de blanchir la peau, et/ou d'éclaircir le teint, et/ou de l'uniformiser, et/ou de l'homogénéiser.

Dans les compositions de l'invention, les trois composés sont présents en une quantité préférentiellement comprise dans une gamme allant de
- 0,01% à 5% pour l'inhibiteur du transfert des mélanosomes, en particulier le nicotinamide,
- 0,01% à 5% pour l'inhibiteur de la tyrosinase dérivé de la vitamine C, en particulier pour l'acide 2-glucoside ascorbique,
- 0,1 à 40 ppm pour l'antagoniste du récepteur MC1-R, en particulier la MELANOSTATINE ^{®}5, préférentiellement 2 à 4 ppm.

Les compostions selon l'invention sont de préférence adaptées à une application topique sur la peau. Les compositions cosmétiques et/ou dermatologiques de l'invention peuvent être utilisées dans un but préventif ou curatif.

Les compositions selon l'invention peuvent en outre comprendre des filtres UVA ou UVB, classiquement utilisés dans des formulations de soin ou de maquillage de jour en cosmétique ou en dermatologie.

Elles peuvent également comprendre des actifs complémentaires tels que des actifs exfoliants, qui ont également un effet éclaircissant sur la peau : par exemple on peut citer les alpha et bêta hydroxyacides.

Les compositions de l'invention peuvent se présenter sous toutes les formes galéniques habituellement employées pour une application topique, et en particulier sous forme d'une solution aqueuse, d'une solution hydroalcoolique ou d'une solution huileuse. Elles peuvent être sous forme d'une émulsion eau-dans-huile, huile-dans-eau, d'une émulsion multiple, d'une dispersion de nanoparticules ou de vésicules lipidiques du type liposomes, d'un gel aqueux, d'un gel huileux, d'un produit anhydre liquide, pâteux ou solide. Cette composition cosmétique et/ou dermatologique peut consister en une formule du type : lotion, gel, crème, mousse, onguent, patch, masque, stick, shampoing, après-shampoing, produit de maquillage.

De façon connue, la composition de l'invention peut contenir également des adjuvants habituels dans le domaine de la cosmétique ou de la dermatologie, tels que par exemple les agents gélifiants, hydrophiles ou lipophiles, les agents émulsionnants, les conservateurs, les anti-oxydants, les charges, les solvants, les parfums, les pigments, les colorants. Elle peut en outre contenir un ou plusieurs autres actifs, qui peuvent être présents dans la même phase que l'association de l'invention, ou, s'il s'agit d'une composition comprenant plusieurs phases, dans une autre phase de la composition. Parmi les autres actifs utilisables dans les compositions de l'invention, on peut citer les agents hydratants, comme la glycérine, les agents anti-vieillissement, tels que les anti-rides, comme par exemples les alpha hydroxyacides, la 7-hydroxy-DHEA, le rétinol. Comme huiles utilisables dans les compositions de l'invention, on peut citer les huiles minérales (huile de vaseline, huile de paraffine), les huiles végétales (huile d'avocat, huile de soja), les huiles d'origine animale (lanoline), les huiles siliconées (cyclométhicone), les huiles de synthèse. La composition peut également contenir d'autres corps gras tels que des alcools gras, des cires (cire de carnauba, cire d'abeille).

Comme agents émulsionnants on peut citer de façon connue : les esters d'acide gras et de polyéthylène glycol, les esters d'acide gras et de glycérine.

### EVALUATION BIOLOGIQUE

Les évaluations sur la mélanogénèse ont été réalisées *in vitro* sur des épidermes reconstruits mélanisés, soumis ou non à une irradiation UV.

Par ailleurs, nous avons montré que l'utilisation de cette association ne conduit pas à un blocage total des mécanismes permettant aux mélanocytes de produire de la mélanine en réponse ou non à une irradiation solaire par exemple. Ainsi, nous avons démontré la réversibilité des effets inhibiteurs de sa composition à l'aide d'une étude réalisée sur des modèles de mélanocytes humains cultivés en monocouche

### Figures :

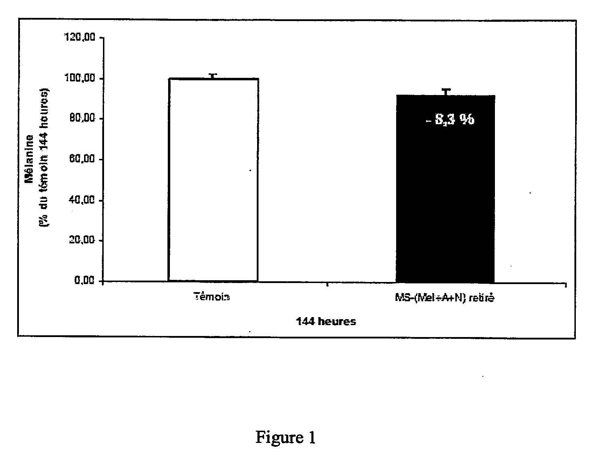

Figure 1 : Réversibilité de l'activité mélano-inhibitrice d'un complexe d'actifs contenant les produits MS-A, MS-N et MS-Mel dans un modèle de mélanocytes humains en culture monocouches ayant subi des irradiations UVB

### I- Etude de la réversibilité de l'effet mélano-inhibiteur de l'association dans un modèle de mélanocytes humains normaux cultivés en monocouche

Le but de cette étude était d'évaluer la réversibilité de l'effet mélano-inhibiteur du complexe contenant les produits MS-A, MS-N et MS-Mel, dans un modèle de mélanocytes humains normaux en culture monocouches.

### I1 - Produits testés

Les produits AA-2G ®, Nicotinamide ® et MELANOSTATINE ® 5 sont respectivement notés MS-A, MS-N et MS-Mel.

### I. 2 - Système d'essai

Des mélanocytes humains normaux ont été obtenus à partir d'un prépuce d'un sujet âgé de 4 ans. Pour la réalisation des essais, ces cellules ont été cultivées jusqu'à l'obtention de monocouches confluentes.

### I. 3 -Inhibiteur de référence

L'inhibiteur de référence utilisé dans cette étude était l'acide kojique à 250 µM.

### I. 4 - Incubation des cellules avec les produits à l'essai

Les mélanocytes ont été incubés pendant 72 heures à 37 °C, sous atmosphère humide et 5 % de CO₂, en absence (contrôle) ou en présence d'acide kojique ou du complexe d'actifs à l'essai contenant MS-A à 2,5.10⁻⁴ M, MS-N à 2,5.10⁻⁴ M et MS-Mel à 10⁻⁴ M. A la fin de cette première période d'incubation, le produit de référence et le complexe d'actifs à l'essai étaient retirés des milieux d'incubation, et les cellules étaient incubées pendant 72 heures supplémentaires en présence de milieu de culture seul. Toutes les 24 heures, les cellules étaient alors irradiées ou non par des UVB à raison de 0,05 J/cm².

### I. 5 - Evaluation des effets

### I. 5. 1 - Dosage de la mélanine

A la fin de la période d'incubation, le contenu intracellulaire en mélanine a été quantifié dans les lysats cellulaires par une mesure spectrophotométrique à 405 nm.

### I. 5. 2 - Dosage des protéines

A la fin de la période d'incubation, les protéines contenues dans les lysats cellulaires ont été quantifiées par la méthode spectrocolorimétrique de Bradford.

### I. 6- Résultats

Après une première période d'incubation en présence du complexe qui conduisait à une inhibition significative de la mélanogénèse (- 24,6 % à T72h, p<0,05), les cellules conservaient leur capacité à produire de la mélanine lorsque les produits étaient enlevés du milieu de culture et ce sans irradiation ou après irradiation UV. En effet, l'inhibition n'est plus que de 9,6% et 8,3% sans irradiation ou après irradiation UV respectivement (Figure 1).

La présente étude a permis de montrer que l'utilisation d'un complexe dépigmentant contenant les produits MS-A, MS-N et MS-Mel, n'entraînait pas de blocage complet et non spécifique des mécanismes impliqués dans la mélanogénèse. En effet, son activité mélano-inhibitrice était tout à fait réversible, puisque des cellules mises en présence de ce complexe pendant 72 heures, conservaient leur capacité à produire de la mélanine lorsque ce dernier était retiré des milieux d'incubation.

### FORMULATION

Dans les exemples, les pourcentages sont des pourcentages en poids.

**Exemple 1 : crème de soin éclaircissante**

| | |
|---|---|
| Arlacel ® 165 | 5,00 |
| Cire d'abeille | 5,00 |
| Huile de paraffine liquide | 5,00 |
| Alcool cétylique | 3,00 |
| AA-2G^{®} | 2,00 |
| Nicotinamide | 2,00 |
| Emulgin ® B2 | 2,00 |
| Sepigel^{®} 305 | 1,60 |
| MELANOSTATINE^{®} 5 | 0,40* |
| Méthylaraben | 0,30 |
| Propylparaben | 0,20 |
| Throméthamine | qsp pH = 6,2 |
| Eau déminéralisée | qsp 100 |

| | |
|---|---|
| (* soit 4 ppm dans la crème) | |

**Exemple 2 : lotion**

| | |
|---|---|
| Glycérine | 3,00 |
| AA-2G^{®} | 2,00 |
| Nicotinamide | 2,00 |
| Pluronic ® PE 6400 | 2,00 |
| Cosmocil CQ^{®} | 0,50 |
| MELANOSTATINE^{®} 5 | 0,40* |
| Parfum | 0,20 |
| Throméthamine | qsp pH = 6,4 |
| Eau déminéralisée | qsp 100 |

| | |
|---|---|
| (* soit 4 ppm dans la lotion) | |

**Exemple 3 : crème de soin « peaux matures »**

| | |
|---|---|
| Arlacel ® 165 | 5,00 |
| Cire d'abeille | 5,00 |
| Huile de paraffine liquide | 6,00 |
| Alcool cétylique | 3,00 |
| AA-2G^{®} | 2,00 |
| Nicotinamide | 2,00 |
| Gatuline^{®} RC | 2,00 |
| Emulgin ^{®} B2 | 2,00 |
| Nuteline^{®} C | 2,00 |
| Sepigel^{®} 305 | 1,80 |
| CM GLUAN P | 1,00 |
| MELANOSTATINE^{®} 5 | 0,40* |
| Méthylaraben | 0,30 |
| Propylparaben | 0,20 |
| α-Lupaline^{®} | 0,10 |
| Throméthamine | Qsp pH = 6,3 |
| Eau déminéralisée | qsp 100 |

| | |
|---|---|
| (* soit 4 ppm dans la crème) | |

## Revendications

1. Association comprenant au moins un antagoniste du récepteur MC1-R, un inhibiteur de la tyrosinase choisi parmi les esters de l'acide ascorbique et un inhibiteur du transfert des mélanosomes vers les kératinocytes, **caractérisée en ce que** ledit antagoniste de récepteur MC1-R est le produit nommé nonapeptide-1 selon la nomenclature INCI

2. Association selon la revendication 1, **caractérisée en ce que** l'inhibiteur de la tyrosinase dérivé de la vitamine C est l'acide 2-glucoside ascorbique.

3. Association selon l'une quelconque des revendications précédentes, **caractérisée en ce que** l'inhibiteur du transfert des mélanosomes vers les kératinocytes est le nicotinamide.

4. Composition cosmétique et/ou dermatologique comprenant une association selon l'une quelconque des revendications précédentes dans un support cosmétiquement ou dermatologiquement acceptable.

5. Composition cosmétique et/ou dermatologique selon la revendication 4, **caractérisée en ce qu'**elle comprend :
- 0,01% à 5% d'inhibiteur du transfert des mélanosomes,
- 0,01% à 5% d'inhibiteur de la tyrosinase choisi parmi les esters de l'acide ascorbique,
- 0,1 à 40 ppm d'antagoniste du récepteur MC1-R.

6. Composition cosmétique et/ou dermatologique selon la revendication 5, **caractérisée en ce qu'**elle comprend :
- 0,01 % à 5% de nicotinamide,
- 0,01% à 5% d'acide 2-glucoside ascorbique.
- 0,1 à 40 ppm de nonapeptide-1.

7. Utilisation cosmétique d'une composition selon l'une quelconque des revendications 4 à 6 pour prévenir et/ou traiter l'apparition d'une hyperpigmentation de la peau et/ou des phanères.

8. Utilisation d'une association selon l'une quelconque des revendications 1 à 3 pour la préparation d'une composition dermatologique destinée à prévenir ou traiter l'apparition d'une hyperpigmentation de la peau et/ou des phanères.

9. Utilisation selon la revendication 7, pour blanchir la peau et/ou éclaircir le teint, et/ou uniformiser le teint, et/ou homogénéiser le teint.

10. Utilisation selon la revendication 8, en vue de prévenir ou traiter les hyperpigmentations régionales dues à une hyperactivité mélanocytaire, sélectionnées parmi :
- les mélasmas idiopathiques, ou ceux qui sont la conséquence d'une contraception oestro-progestative ;
- les hyperpigmentations localisées par hyperactivité et prolifération mélanocytaire bénigne, telles que les taches de rousseur, les taches solaires ou les taches pigmentaires séniles, dites lentigo actiniques ;
- les hyperpigmentations ou dépigmentations accidentelles.

## Patentansprüche

1. Verbindung, die wenigstens einen Antagonisten des Empfängers MC1-R, einen Inhibitor der Tyrosinase, der aus den Estern der Ascorbinsäure ausgewählt ist, und einen Inhibitor des Transfers der Melanosome auf die Keratinozyten umfasst, **dadurch gekennzeichnet, dass** der genannte Rezeptorantagonist MC1-R das gemäß der Nomenklatur INCI Nonapeptid-1 genannte Produkt ist.

2. Verbindung gemäß Anspruch 1, **dadurch gekennzeichnet, dass** der aus dem Vitamin C abgeleitete Inhibitor der Tyrosinase Askorbinsäure-2-Glukosid ist.

3. Verbindung gemäß einem der voranstehenden Ansprüche, **dadurch gekennzeichnet, dass** der Inhibitor des Transfers der Melanosome auf die Keratinozyten Nikotinamid ist.

4. Kosmetische und/oder dermatologische Zusammensetzung, die eine Verbindung gemäß einem der voranstehenden Ansprüche in einem kosmetisch oder dermatologisch akzeptablen Träger umfasst.

5. Kosmetische und/oder dermatologische Zusammensetzung gemäß Anspruch 4, **dadurch gekennzeichnet, dass** sie:
- 0,01 % bis 5 % des Inhibitors des Transfers der Melanosome,
- 0,01 % bis 5 % des Inhibitors der aus den Estern der Ascorbinsäure ausgewählten Tyrosinase,
- 0,1 bis 40 ppm des Antagonisten des Rezeptors MC1-R umfasst.

6. Kosmetische und/oder dermatologische Zusammensetzung gemäß Anspruch 5, **dadurch gekennzeichnet, dass** sie:
- 0,01 % bis 5 % Nikotinamid,
- 0,01 % bis 5 % Askorbinsäure-2-Glukosid
- 0,1 % bis 40 ppm Nonapeptid-1 umfasst.

7. Kosmetische Verwendung einer Zusammensetzung gemäß einem der Ansprüche 4 bis 6 zur Vorbeugung und/oder Behandlung des Auftretens einer Hyperpigmentierung der Haut und/oder von Hautanhangsgebilden.

8. Verwendung einer Verbindung gemäß einem der Ansprüche 1 bis 3 für die Zubereitung einer dermatologischen Zusammensetzung, die zur Vorbeugung oder Behandlung des Auftretens einer Hyperpigmentierung der Haut und/oder von Hautanhangsgebilden der Haut bestimmt ist.

9. Verwendung gemäß Anspruch 7 zum Aufhellen der Haut und/oder zum Aufhellen des Teints und/oder zum Uniformisieren des Teints und/oder zum Homogenisieren des Teints.

10. Verwendung gemäß Anspruch 8 zur Vorbeugung oder Behandlung der regionalen Hyperpigmerttierungen, die auf eine melanozytäre Hyperaktivität zurückzuführen sind und aus:
- Idiopathischen Melasmen oder denen, die die Folge einer östro-progestativen Schwangerschaftsverhütung sind;
- Iokalisierten Hyperpigmentierungen aufgrund von Hyperaktivität und gutartiger melanozytärer Proliferation, wie z. B. Sommersprossen, Sonnenflecken oder Altersflecken, bezeichnet als Lentigo Actinica;
- ungewollten Hyperpigmentierungen oder Depigmentierungen ausgewählt sind.

## Claims

1. Association including at least one MC1-R receptor antagonist, one tyrosinase inhibitor chosen from among the esters of ascorbic acid, and one inhibitor of transfer of melanosomes to keratinocytes, **characterized by** the fact that the said MC1-R receptor antagonist is the product named nonapeptide-1 according to the INCI nomenclature.

2. Association in accordance with claim 1, **characterized by** the fact that the tyrosinase inhibitor derived from vitamin C is ascorbic 2-glucoside acid.

3. Association in accordance with any one of the preceding claims, **characterized by** the fact that the inhibitor of transfer of melanosomes to keratinocytes is nicotinamide.

4. Cosmetic and/or dermatological composition including an association in accordance with any one of the preceding claims, in a cosmetically- or dermatologically-acceptable medium.

5. Cosmetic and/or dermatological composition in accordance with claim 4, **characterized by** the fact that it includes:
- 0.01% to 5% of melanosome transfer inhibitor;
- 0.01% to 5% of tyrosinase inhibitor chosen from among the esters of ascorbic acid;
- 0.1 to 40 ppm of MC 1-R receptor antagonist.

6. Cosmetic and/or dermatological composition in accordance with claim 5, **characterized by** the fact that it includes:
- 0.01% to 5% of nicotinamide;
- 0.01% to 5% of ascorbic 2-glucoside acid;
- 0.1 to 40 ppm of nonapeptide-1.

7. Cosmetic use of a composition according to any one of claims 4 to 6, to prevent and/or treat the appearance of a hyperpigmentation of the skin and/or dermaskeleton.

8. Use of an association in accordance with any one of claims 1 to 3 for the preparation of a dermatological composition intended to prevent or treat the appearance of a hyperpigmentation of the skin and/or dermaskeleton.

9. Use in accordance with claim 7, for whitening the skin, and/or lightening the tone, and/or uniformizing the tone, and/or homogenizing the tone.

10. Use in accordance with claim 8, in view of preventing or treating regional hyperpigmentations due to a melanocytar hyperactivity, from among:
- idiopathic melasmas, or those that have the consequence of an oestro-progestative contraception;
- localized hyperpigmentations due to benign melanocytic proliferation and hyperactivity, such as freckle marks, solar lentigos, or senile pigmentation marks referred to as lentigo senilis;
- accidental hyperpigmentations or depigmentations.
